# EUROPEAN PATENT APPLICATION

(11) **EP 2 965 703 A1**
(43) Date of publication of application: **13.01.2016**
(21) Application number: 14760967.1
(22) Date of filing: 28.02.2014
(51) Int. Cl.: A61B 17/88, A61B 17/56, A61M 25/00

(54) **CANNULA WITH ADJUSTABLE CORNER FOR DELIVERING BONE FILLER**

(30) Priority: 04.03.2013 CN 201310067120
(71) Applicant: Ningbo Hicren Biotechnology Co. Ltd., Ningbo, Zhejiang 315336 (CN)
(72) Inventor: LV, Shiwen, Ningbo Zhejiang 315336 (CN); WANG, Yu, Ningbo Zhejiang 315336 (CN); MAO, Keya, Ningbo Zhejiang 315336 (CN); XIN, Chaohua, Ningbo Zhejiang 315336 (CN)
(74) Representative: Fiussello, Francesco
(86) International application number: PCT/CN2014/072686
(87) International publication number: WO 2014/135029

(57) **Abstract**

Disclosed is a steerable cannula for delivering bone filler, comprising a delivery pipe (10), a delivery handle (30) fixedly connected to the proximal end of the delivery pipe (10), a sheath-core (20) slidably inserted into the delivery pipe (10) and a sheath-core handle (40) fixedly connected to the proximal end of the sheath-core (20). Distal portion of the delivery pipe (10) is a flexible pipe (11) and proximal portion thereof is a rigid pipe (12). The sheath-core (20) has a cavity. Distal portion of the sheath-core (20) is a flexible segment (21) and the proximal portion thereof is a rigid segment (23). One or more pull threads (23) are provided in the cavity of the sheath-core (20), wherein distal ends of the pull thread (23) are fixedly connected to the distal end of the flexible segment (21) of the sheath-core (20) and proximal end thereof is connected to a drawing means arranged on the sheath-core handle (40) to drive the flexible segment (21) of the distal end of the sheath-core (20) to bend. The distal end of the steerable cannula for delivering bone filler can not only bend, but also the degree of bending thereof can be freely controlled by a doctor, making the bone filler distribution more uniform and facilitating the success of an operation.

## Description

### Cross Reference to Related Applications

This application claims the benefit of priority of Chinese Patent Application No. 201310067120.9, filed on March 4, 2013, entitled "STEERABLE CANNULA FOR DELIVERING BONE FILLER", the entire contents of which are incorporated herein by reference.

### Technical Field

The present disclosure relates to the field of medical instruments, more particularly, to a steerable cannula for delivering the bone filler used in PVP (Percutaneous Vertebroplasty) or PKP (Percutaneous Kyphoplasty).

### Background

As the vertebral compression fracture caused by osteoporosis commonly occurs among the aged, with the population aging becoming more serious, the number of patients suffering from this disease is growing fast, and PVP or PKP plays an irreplaceable role in treating this kind of disease. Nowadays, the cannula for delivering the bone filler is widely used in the vertebral plasty. Under the monitoring of imaging equipment, the bone filler, such as the bone cement, is injected into the clearances between the cancellous bones of the vertebra with the help of external forces, so as to restore the height of the vertebral body, improve the strength and the stability of the vertebral body, relieve the pains of patients in the back and in the waist, and cure the vertebral compression fracture caused by osteoporosis.

The distal end of the cannula for delivering the bone filler, which is used in vertebral plasty (PVP or PKP) in China presently, is straight and inflexible. During the operation, it is commonly to inject fillers symmetrically into the vertebral body to keep a biomechanical balance of the vertebral body. The traditional cannula for delivering the bone filler is only capable of delivering the bone filler from the same side into the ipsilateral vertebral body, so each segment of the vertebral body should be punctured twice before injecting the bone filler, which makes the operation last for a longer time, causes greater injuries to the patient and higher incidence rate of complications, and increases economic burden on the patient. The deficiency above is more serious to patients with many vertebral body lesions.

Concerning the deficiency above, the OSSEON Company produced the SB-series of cannula for delivering the bone cement, said cannula has an adjustable bendable segment at its distal end, but this segment is formed through laser cutting. Thus the cannula has the following deficiencies:
1. The bendable segment at the distal end of the cannula is bended through pulling the adjusting thread backward by force. However, the adjusting thread is provided in the injection cavity for the bone cement, occupying the inner space of the cavity, which reduces the space of the injection passage, and prolongs the injecting time, thereby causing it more difficult to inject the bone cement.
2. As the adjusting thread is provided in the injection cavity for the bone cement, and the bone cement is in a viscous state when being injected, the bone cement will adhere to the adjusting thread, blocking the whole injection cavity and affecting the adjustment on the bendable segment by the adjusting thread.
3. As the adjustable bendable segment is formed through cutting a metal tube by laser, the adjustable bendable segment is apt to contort during operation, as a result the cannula for delivering the bone cement cannot be removed from the vertebral body.

### Summary

The present invention aims at providing a steerable cannula for delivering the bone filler which is flexible to use, convenient to operate and simple in structure, and which has lower production cost and better safety performance. In the present invention, the distal end of the delivery pipe is designed to be a flexible segment, and a sheath-core having a steerable distal end is inserted into the delivery pipe, so that the distal end of the delivery pipe can be bended and the operator can adjust the bending angle of the distal end of the delivery pipe by pulling a pull thread inside the sheath-core.

The object of the present invention is achieved by the following technical scheme:
A steerable cannula for delivering the bone filler includes a delivery pipe, a delivery handle fixedly connected to a proximal end of the delivery pipe, a sheath-core slidably inserted into the delivery pipe and a sheath-core handle fixedly connected to a proximal end of the sheath-core;
   wherein, a distal portion of the delivery pipe is a flexible pipe and a proximal portion of the delivery pipe is a rigid pipe; the sheath-core includes a tube having a cavity, a distal portion of the sheath-core is a flexible segment and a proximal portion of the sheath-core is a rigid segment; one or more pull threads are provided inside the cavity of the sheath-core; a distal end of each pull thread is fixedly connected to the distal end of the flexible segment of the sheath-core; a proximal end of each pull thread is connected to a drawing means arranged on the sheath-core handle, so as to drive the flexible segment at the distal end of the sheath-core to bend, and further to drive the distal end of the delivery pipe to bend.

The purposes of the present invention can be further achieved by the following technical solutions:

Preferably, the sheath-core has a single cavity or more cavities; in case of more cavities, one strengthening wire is provided inside one of the cavities; a proximal end of the strengthening wire is fixedly connected to the sheath-core handle.

Preferably, the drawing means includes a sliding groove arranged longitudinally along an axle direction of the sheath-core handle and a sliding block arranged in the sliding groove; the proximal end of each pull thread is connected to the sliding block; the pull thread is configured to be drawn through sliding the sliding block arranged in the sliding groove towards the proximal end, and the flexible segment of the distal end of the sheath-core is further driven to bend with a bending angle ranged from zero degree to 120 degrees.

Preferably, a cover plate is fixedly attached to the distal end of the sheath-core handle; the cover plate is provided with a pull thread hole; and each pull thread goes through the pull thread hole and is fixedly connected to the sliding block.

Preferably, the drawing means is a knob arranged on the sheath-core handle; the proximal end of each pull thread is fixedly connected to the knob; the pull thread is configured to be drawn by turning the knob, and the flexible segment at the distal end of the sheath-core is further driven to bend with a bending angle ranged from zero degree to 120 degrees..

Preferably, the pull thread has a diameter ranged from 0.05 mm to 2 mm.

Preferably, the distal end of the delivery pipe is closed; the distal end of the delivery pipe is provided with one or more holes at its side wall; the distal end of the delivery pipe is arc-shaped, flat-shaped or sharp-shaped; and the delivery handle is provided with a Luer joint for connecting a syringe of bone filler.

Preferably, the distal end of the delivery pipe is open; the distal end of the sheath-core is closed, and the distal end of the sheath-core is arc-shaped, flat-shaped or sharp-shaped.

Preferably, the distal end of the delivery pipe or the distal end of the sheath-core is sealed by a tapered metal head.

Preferably, the flexible segment of the sheath-core is provided with several grooves at its tube wall.

As compared with the prior art, the present invention has the following features and improvements:
Firstly, the steerable cannula for delivering the bone filler of the present invention includes the delivery pipe and the sheath-core, the distal end of the delivery pipe is a flexible segment, therefore the bending angle can be adjusted randomly. The proximal end of the delivery pipe is a rigid segment, ensuring sufficient pushing force. The distal end of the sheath-core is provided with an adjustable flexible segment, the proximal end of the sheath-core is connected to the sheath-core handle, the sheath-core includes a tube having a single or more cavities, one or more pull threads are provided in the cavity; the distal end of the pull thread is fixedly connected to the adjustable flexible segment, the proximal end of the pull thread is connected to the drawing means arranged on the sheath-core handle. The bending angle of the distal end of the sheath-core is adjusted through pulling the pull thread by the drawing means, enabling the bone filler to be delivered to any position on opposite sides of the vertebral body, thereby the present invention can reduce the number of punctures during the vertebral plasty, relieve the sufferings of the patients, make the operator operate more flexibly and reduce the incidence of complications. A strengthening wire is further provided in another cavity of the sheath-core, which can increase the strength of the sheath-core. The flexible segment restores to the initial state when the pull thread is released.
Secondly, the adjusting pull thread of the present invention is arranged in the cavity of the sheath-core and is separated from the injection cavity for the bone cement, therefore the adjusting pull thread will not affect the use of the cannula for delivering the bone cement and will not affect the delivery of the bone cement.
Thirdly, the sheath-core of the cannula for delivering the bone filler of the present invention is inserted into the delivery pipe in use. Even if the adjustable bendable segment of the sheath-core is deformed during operation, the sheath-core will not be stuck in the working sleeve because of the protection of the delivery pipe, avoiding the situation that the sheath-core cannot be withdrawn.
Fourthly, the distal end of the delivery pipe of the cannula for delivering the bone filler is flexible, which can reduce the damage to the patient by rigid materials, and relieve the postoperative sufferings of the patients.

Finally, preferably, in the cannula for delivering the bone filler of the present invention, the distal end of the delivery pipe or the distal end of the sheath-core is sharp-shaped, so that the cannula for delivering the bone filler can smoothly shove into and squeeze the cancellous bone in the vertebral body, and arrive at the predesigned position more easily.

### Brief Description of Drawings

Fig.1 is a structural schematic diagram of the first embodiment of the present invention;
Fig.2 is a structural schematic diagram illustrating the delivery pipe and the delivery handle of the first embodiment of the present invention;
Fig.3 is a structural schematic diagram illustrating the distal end of the delivery pipe of the first embodiment of the present invention; wherein, Fig.3a is a structural schematic diagram illustrating an arc-shaped distal end; Fig.3b is a structural schematic diagram illustrating a sharp-shaped distal end; and Fig.3c is a structural schematic diagram illustrating a flat-shaped distal end;
Fig.4 is a structural schematic diagram illustrating the distal end of the delivery pipe of the first embodiment of the present invention; wherein, Fig.4a is a structural schematic diagram illustrating a distal end of the delivery pipe which is naturally transitional and closed; and Fig.4b is a structural schematic diagram illustrating a distal end of the delivery pipe which is fixed with a sealing head;
Fig.5 is a structural schematic diagram illustrating the sheath-core and the sheath-core handle of the first embodiment of the present invention;
Fig.6 is a structural schematic diagram illustrating the sheath-core of the first embodiment of the present invention;

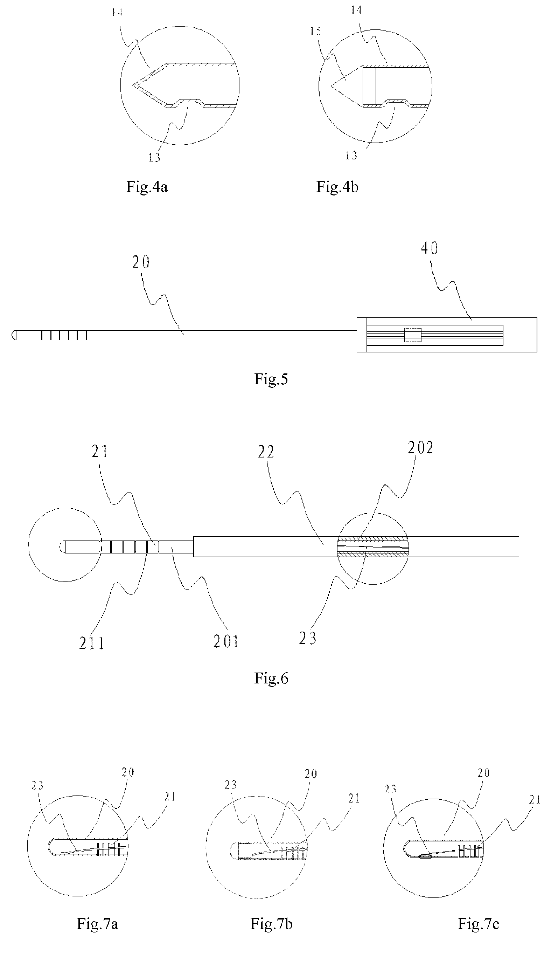

Fig.7 is a structural schematic diagram illustrating how the pull thread arranged in the sheath-core is fixedly connected with the flexible segment of the sheath-core according to the first embodiment of the present invention; wherein, Fig.7a is a schematic diagram illustrating that the pull thread is connected to the flexible segment of the sheath-core through welding; Fig.7b is a schematic diagram illustrating that the pull thread is connected to the flexible segment of the sheath-core through adhesive bonding; and Fig.7c is a schematic diagram illustrating that the pull thread is connected with the flexible segment of the sheath-core through knotting;
Fig.8 is a structural schematic diagram illustrating the sheath-core handle of the first embodiment of the present invention; wherein, Fig.8a is a sectional view of the sheath-core handle; and Fig.8b is an exploded view of the sheath-core handle;
Fig.9 is a structural schematic diagram illustrating the pull thread that is composed of metal materials and medical polymer materials according to the first embodiment of the present invention; wherein, Fig.9a is a structural schematic diagram illustrating the pull thread that is composed of one metal wire and polymer materials; and Fig.9b is a structural schematic diagram illustrating the pull thread that is composed of several metal wires and polymer materials;
Fig.10 is a schematic diagram illustrating a first working state of the cannula for delivering the bone filler according to the present disclosure;
Fig.11 is a schematic diagram illustrating a second working state of the cannula for delivering the bone filler according to the present disclosure;
Fig.12 is a schematic diagram illustrating a third working state of the cannula for delivering the bone filler according to the present disclosure;
Fig.13 is a schematic diagram illustrating a fourth working state of the cannula for delivering the bone filler according to the present disclosure;
Fig.14 is a schematic diagram illustrating the working state of the cannula for delivering the bone filler according to the present disclosure, wherein the sheathe-core has been moved;
Fig. 15 is a structural schematic diagram of the second embodiment of the present invention;
Fig.16 is a structural schematic diagram illustrating the distal end of the sheath-core according to the second embodiment of the present invention;
Fig.17 is a structural schematic diagram illustrating the delivery pipe according to the second embodiment of the present invention;
Fig.18 is a structural schematic diagram of the third embodiment of the present invention;
Fig.19 is a structural schematic diagram illustrating the sheath-core handle according to the third embodiment of the present invention;
Fig.20 is a structural schematic diagram illustrating the sheath-core according to the fourth embodiment of the present invention;
Fig.21 is a structural schematic diagram illustrating the sheath-core with multiple cavities according to the fourth embodiment of the present invention; wherein, Fig.21a is a longitudinal sectional view of the sheath-core; and Fig.21b is a cross sectional view of the sheath-core.

### Detailed Description of Disclosed Embodiments

### The First Embodiment

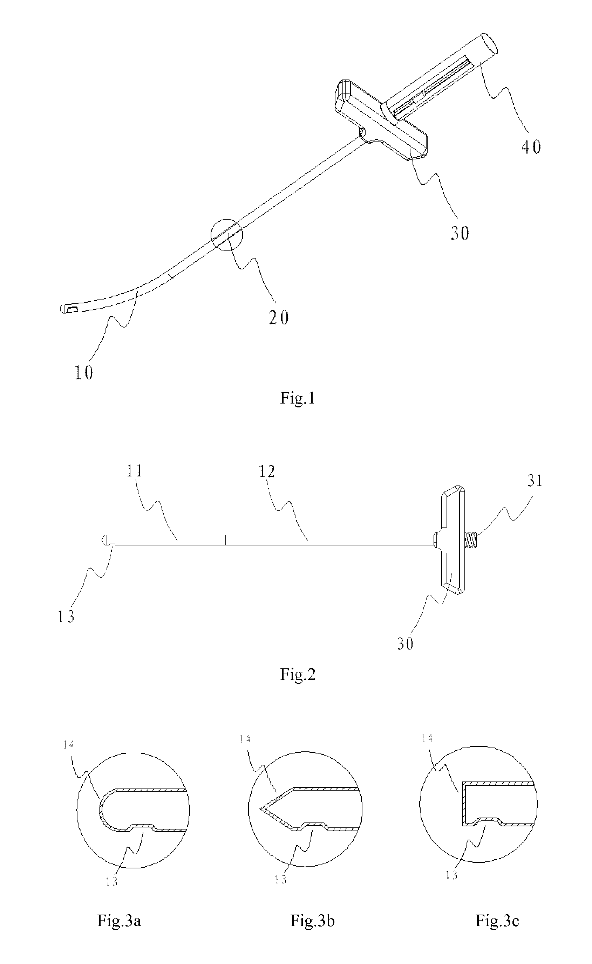

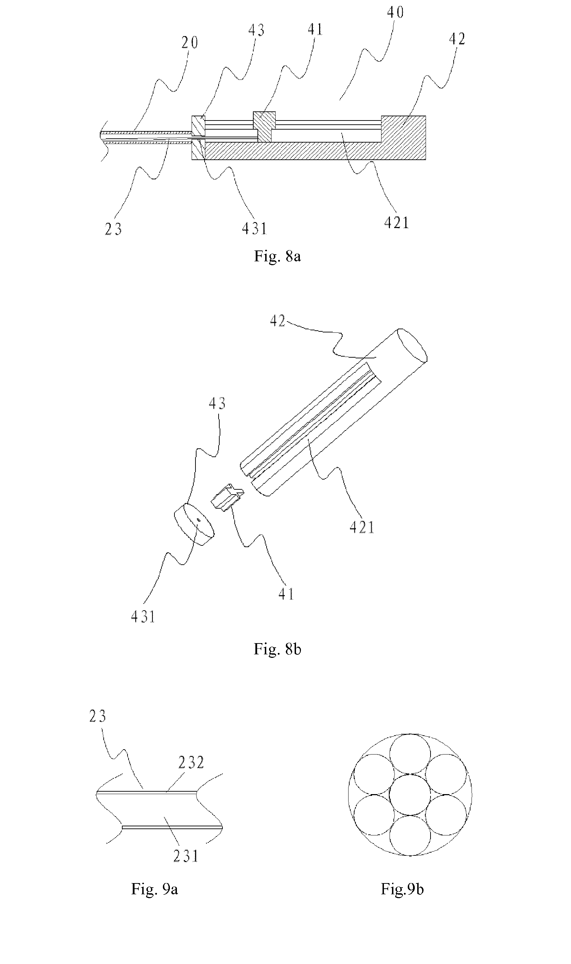

As shown in Figures 1-9, a steerable cannula for delivering the bone filler includes a delivery pipe 10, a delivery handle 30 fixedly connected to the proximal end of the delivery pipe 10, a sheath-core 20 slidably inserted into the delivery pipe 10 and a sheath-core handle 40 fixedly connected to the proximal end of the sheath-core 20. Wherein, the distal portion of the delivery pipe 10 is a flexible pipe 11 and the other portion thereof is a rigid pipe 12. The distal end 14 of the delivery pipe 10 is closed, being arc-shaped, sharp-shaped or flat-shaped, as shown in Fig.3. The distal end 14 of the delivery pipe 10, as shown in Fig.4a, is naturally transitional and closed, or, as shown in Fig.4b, is fixedly connected with a sealing head 15 through welding, adhesive bonding, or screw connecting, and so on. The distal end of the delivery pipe 10 is provided with one or more holes 13 at its side wall. The proximal end of the flexible pipe 11 is fixedly connected with the distal end of the rigid pipe 12 through adhesive bonding, hot welding, rivet connecting or screw connecting, and so on. The proximal end of the rigid pipe 12 is fixedly connected to the delivery handle 30. The delivery handle 30 is provided with a Luer joint 31 for connecting the syringe of bone filler. As shown in Fig.5 and Fig.6, the distal portion of the sheath-core 20 is a flexible segment 21 with an adjustable bending angle, and the proximal portion thereof is a rigid segment 22. The proximal end of the rigid pipe 22 is fixedly connected to the sheath-core handle 40. The adjustable bending angle of the flexible segment 21 is ranged from zero degree to 120 degrees. The distal portion of the sheath-core 20 is provided with grooves 211 at its tube wall so as to form the flexible segment 21 with an adjustable bending angle. The sheath-core 20 is a tube having a single cavity, and one or more pull threads 23 are disposed inside the cavity. As shown in Fig.9a and Fig.9b, the pull thread 23 is a non-elastic metal wire or a non-elastic pull thread made of medical polymer materials, or composed of metal wires 231 and the medical polymer materials 232. The distal end of the pull thread 23 is fixedly connected to the distal end of the flexible segment 21 of the sheath-core 20 through welding, knotting or adhesive bonding, and so on. As shown in Fig.7a and Fig.7b, the distal end of the pull thread 23 is welded or adheres to the inner wall of the distal end of the flexible segment 21 of the sheath-core 20. As shown in Fig.7c, the distal end of the flexible segment 21 of the sheath-core 20 is provided with a hole at its side wall. The pull thread 23 goes through the hole and is knotted so as to be attached to the distal end of the flexible segment 21. As shown in Fig.8a and Fig.8b, a sliding groove 42 is disposed along the longitudinal direction of the sheath-core handle 40, and a sliding block 41 is arranged in the sliding groove 42. The proximal end of the pull thread 23 is connected with the sliding block 41. The sliding block 41 is capable of sliding in the sliding groove 42, so as to draw the pull thread 23 to bend the flexible segment 21 with an adjustable bending angle. In a preferred embodiment, a cover plate 43 is fixedly attached to the distal end of the sheath-core handle 40. The cover plate 43 is provided with a pull thread hole 431, the pull thread 23 goes through the pull thread hole and is fixedly connected with the sliding block 41.

In the embodiment above, the flexible pipe 11 of the delivery pipe 10 is made of medical polymer materials including but not limited to polyurethane, polypropylene, polyethylene, polycarbonate, ABS resin or modified nylon, and so on (polyethylene is preferred). The rigid pipe 12 is a metal tube (preferably, 304 stainless steel) or a medical polymer tube with higher strength. The delivery handle 30 and the sheath-core handle 40 are made of medical polymer materials, preferably made of polycarbonate. The sliding block 41 is made of medical polymer materials or metal materials (preferably made of 304 stainless steel). The sheath-core 20 is made of a metal tube 201 or is composed of a metal tube 201 and a medical polymer material tube 202.

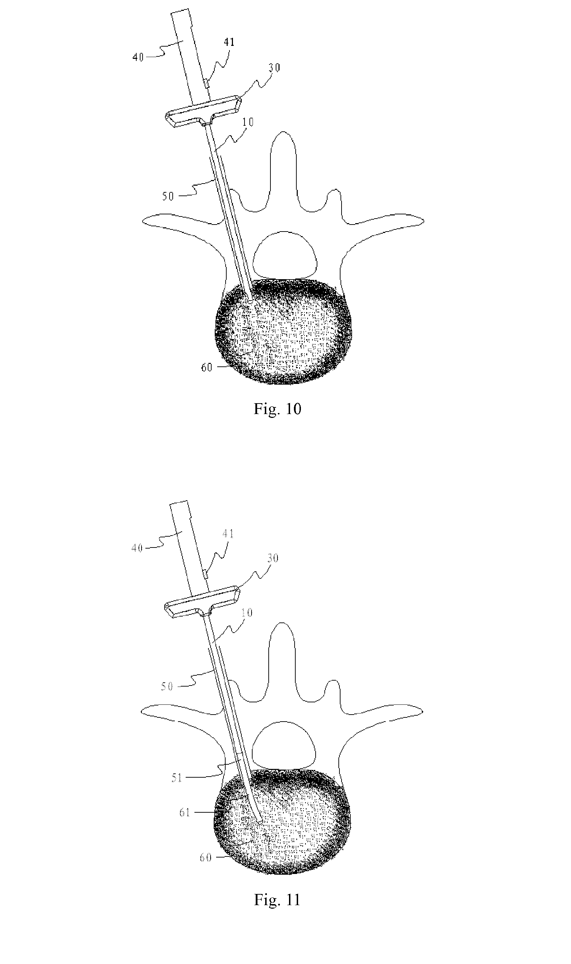

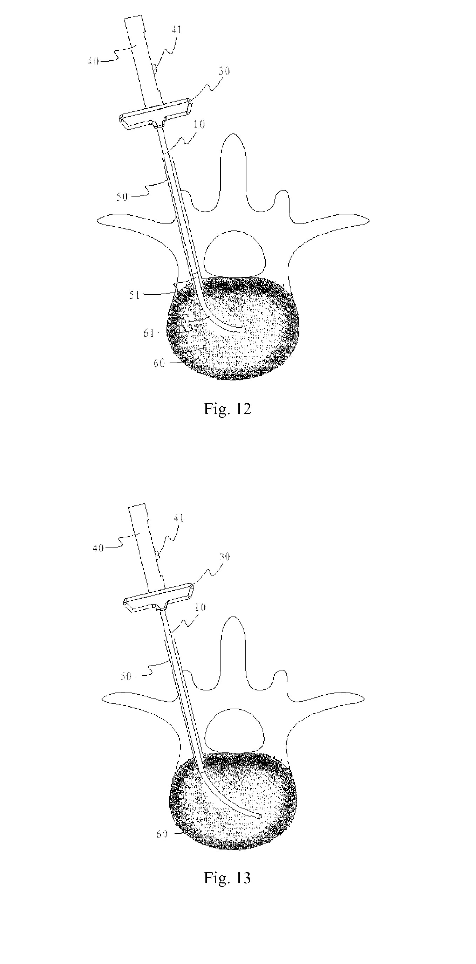

As shown in Figures 10-14, the operation process of the percutaneous vertebroplasty (PVP) or the percutaneous kyphoplasty (PKP) is as follows:
Firstly, locate the diseased vertebra with C-arm fluoroscopy imaging or CT monitoring; then a needle is punctured through percutaneous vertebral pedicles, creating an operation access; produce angiography to reflect the conditions inside the vertebral body; then insert the cannula for delivering the bone filler into the working sleeve 50, until the distal end of the sheath-core 20 reaches the distal end of the working sleeve 50.
Secondly, push the cannula for delivering the bone filler forward by 5 to 10 mm. At this time the flexible pipe 11 of the delivery pipe 10 and the distal end 61 of the flexible segment 21 of the sheath-core 20 enter inside the vertebral body 60, and the proximal end 51 of the flexible segment 21is still inside the working sleeve 50.
Thirdly, slide the sliding block 41 so as to make the distal end 61 of the flexible segment 21 of the sheath-core 20, which has entered inside the vertebral body, bend for 5 to 10 degrees. At this time, the proximal end 51 of the flexible segment 21 of the sheath-core 20, which is sleeved inside the working sleeve 50, remains straight because of the restriction of the working sleeve 50.
Fourthly, keep pushing the cannula for delivering the bone filler until the flexible pipe 11 and the flexible segment 21 both enter inside the vertebral body 60 completely.
Fifthly, release the sliding block 41 so as to make the pull thread 23 loose. Withdraw the sheath-core 20, and the flexible segment 21 of the sheath-core 20 will be withdrawn along the internal passage of the vertebral body 60 and along the working sleeve 50. The flexible pipe 11 of the delivery pipe 10 remains curving because of the restriction of the internal passage of the vertebral body 60.
Finally, connect the syringe of bone filler to the Luer joint 31 of the delivery handle 30, and inject the bone filler into the vertebral body 60 through the delivery pipe 10.

Since the whole process of the operation is imaged or monitored, according to the actual conditions of the patient, the doctor can determine the specific operating steps, and determine the pushing distance and bending angle in each of the steps, so that the bone filler can be delivered to any position inside the vertebral body 60.

### The Second Embodiment

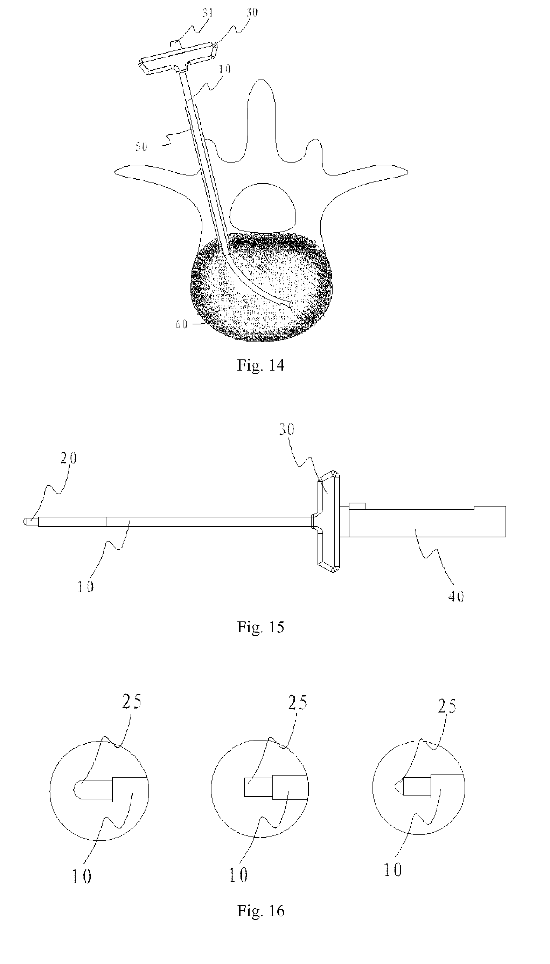

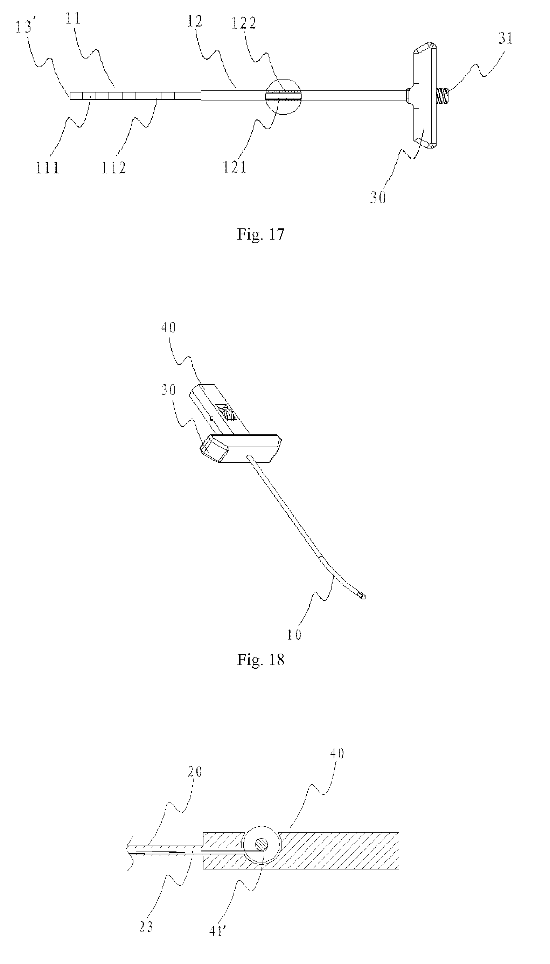

As shown in Figures 15-17, a steerable cannula for delivering the bone filler includes a delivery pipe 10, a delivery handle 30 fixedly connected to the proximal end of the delivery pipe 10, a sheath-core 20 slidably inserted into the delivery pipe 10 and a sheath-core handle 40 fixedly connected to the proximal end of the sheath-core 20. Wherein, the distal portion of the delivery pipe 10 is a flexible pipe 11 and the other portion thereof is a rigid pipe 12. The distal end of the delivery pipe 10 is provided with an opening 13. The distal end of the delivery pipe 10 is provided with one or more holes at its side wall. The proximal end of the flexible pipe 11 is fixedly connected with the distal end of the rigid pipe 12 through adhesive bonding, hot welding, rivet connecting or screw connecting, and so on. The proximal end of the rigid pipe 12 is fixedly connected to the delivery handle 30. The delivery handle 30 is provided with a Luer joint 31 for connecting the syringe of bone filler. The distal portion of the sheath-core 20 is a flexible segment 21 with an adjustable bending angle, and the proximal portion thereof is a rigid segment 22. The proximal end of the rigid pipe 22 is fixedly connected to the sheath-core handle 40. The adjustable bending angle of the flexible segment 21 is ranged from zero degree to 120 degrees. As shown in Fig.16, the distal end 25 of the sheath-core 20 is closed, being sharp-shaped, arc-shaped, or flat-shaped. The sheath-core 20 is slidably inserted in the delivery pipe 10, and the distal end 25 of the sheath-core 20 extends out of the opening 13 of the distal end of the delivery pipe 10. The distal portion 25 of the sheath-core 20 is provided with grooves 211 at its tube wall so as to form the flexible segment 21 with an adjustable bending angle. The sheath-core 20 is a tube with a single cavity, and one or more pull threads 23 are disposed inside the cavity. The distal end of the pull thread 23 is fixedly connected to the distal end of the flexible segment 21 of the sheath-core 20 through welding, knotting or adhesive bonding, and so on. A sliding groove 42 is disposed along the longitudinal direction of the sheath-core handle 40, and a sliding block 41 is arranged in the sliding groove 42. The proximal end of the pull thread 23 is connected with the sliding block 41. The sliding block 41 is capable of sliding in the sliding groove 42, so as to draw the pull thread 23 to bend the flexible segment 21 with an adjustable bending angle.

The flexible pipe 11 of the delivery pipe 10 is composed of medical polymer materials 111 and a metal tube 112. The rigid pipe 12 of the delivery pipe 10 is composed of medical polymer materials 121 and a metal tube 122. The sheath-core 20 is made of a metal tube, and the delivery handle 30 and the sheath-core handle 40 are made of medical polymer materials, preferably made of polycarbonate.

### The Third Embodiment

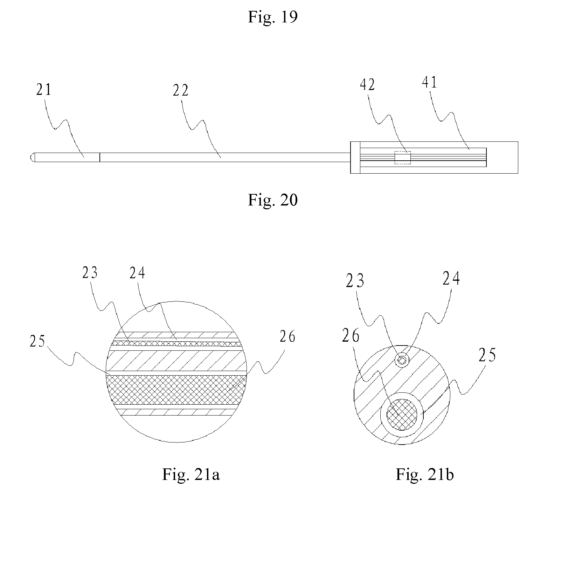

As shown in Fig.2, Fig.5, Fig.6, Fig.7, Fig.18 and Fig.19, a steerable cannula for delivering the bone filler includes a delivery pipe 10, a delivery handle 30 fixedly connected to the proximal end of the delivery pipe 10, a sheath-core 20 slidably inserted into the delivery pipe 10 and a sheath-core handle 40 fixedly connected to the proximal end of the sheath-core 20. Wherein, the distal portion of the delivery pipe 10 is a flexible pipe 11 and the other portion thereof is a rigid pipe 12. The distal end of the delivery pipe 10 is closed, and is provided with one or more holes 13 at its side wall. The proximal end of the flexible pipe 11 is fixedly connected with the distal end of the rigid pipe 12 through adhesive bonding, hot welding, rivet connecting or screw connecting, and so on. The proximal end of the rigid pipe 12 is fixedly connected to the delivery handle 30. The delivery handle 30 is provided with a Luer joint 31 for connecting the syringe of bone filler. The distal portion of the sheath-core 20 is provided with grooves 211 at its tube wall so as to form the flexible segment 21 with an adjustable bending angle. The adjustable bending angle of the flexible segment 21 is ranged from zero degree to 120 degrees. The proximal portion of the sheath-core 20 is a rigid segment 22, and the proximal end of the rigid segment 22 is fixedly connected to the sheath-core handle 40. The sheath-core handle 40 is provided with a knob 41. The sheath-core 20 is a tube with a single cavity, and one or more pull threads 23 are disposed inside the cavity. As shown in Fig.9a, the pull thread 23 is a metal wire or a pull thread made of medical polymer materials, or is composed of a metal wire 231 and the medical high poly materials 232. The distal end of the pull thread 23 is fixedly connected to the distal end of the flexible segment 21 of the sheath-core 20 through welding, knotting or adhesive bonding, and so on. The proximal end of the pull thread 23 is connected with the knob 41 of the sheath-core handle 40. As shown in Fig.19, the knob 41 can be turned to draw the pull thread 23 so as to bend the flexible segment 21 with an adjustable bending angle.

### The Fourth Embodiment

As shown in Fig.1, Fig.2, Fig.20 and Fig.21, a steerable cannula for delivering the bone filler includes a delivery pipe 10, a delivery handle 30 fixedly connected to the proximal end of the delivery pipe 10, a sheath-core 20 slidably inserted into the delivery pipe 10 and a sheath-core handle 40 fixedly connected to the proximal end of the sheath-core 20. Wherein, the distal portion of the delivery pipe 10 is a flexible pipe 11 and the other portion thereof is a rigid pipe 12. The distal end of the delivery pipe 10 is closed, and is provided with one or more holes 13 at its side wall. The proximal end of the flexible pipe 11 is fixedly connected with the distal end of the rigid pipe 12 through adhesive bonding, hot welding, rivet connecting or screw connecting, and so on. The proximal end of the rigid pipe 12 is fixedly connected to the delivery handle 30. The delivery handle 30 is provided with a Luer joint 31 for connecting the syringe of bone filler. The distal portion of the sheath-core 20 is a flexible segment 21 and the proximal portion thereof is a rigid segment 22. The sheath-core 20 is a tube with a first cavity 24 and a second cavity 25, one or more pull threads 23 are disposed inside the first cavity 24, and one strengthening wire 26 is disposed inside the second cavity 25. The strengthening wire 26 is a NiTinol wire, the pull threads 23 are metal wires or made of medical polymer materials. The distal end of the pull thread 23 is fixedly connected to the distal end of the flexible segment 21 of the sheath-core 20 through welding, knotting or adhesive bonding, and so on. A sliding groove 42 is disposed along the longitudinal direction of the sheath-core handle 40, and a sliding block 41 is arranged in the sliding groove 42. The proximal end of the pull thread 23 is connected with the sliding block 41. The sliding block 41 is capable of sliding in the sliding groove 42, so as to draw the pull thread 23 to bend the flexible segment 21 with an adjustable bending angle. The distal end of the strengthening wire 26 is fixedly connected with the distal end of the second cavity 25 of the sheath-core 20; alternatively, the distal end of the strengthening wire 26 is inserted into the second cavity 25 of the sheath-core 20 and extends to the distal end of the second cavity 25. The strengthening wire 26 performs the effect of increasing the strength of the sheath-core 20, and makes the flexible segment 21 restore to the initial state when the pull thread 23 is released.

It should be understood by those skilled in the art that, what described above are preferred embodiments of the present invention, they are not intended to limit the present disclosure, various modifications, replacements and improvements may be made therein without departing from the spirits and principles of the present invention, and should be covered by the scope of the present invention.

## Claims

1. A steerable cannula for delivering bone filler, comprising a delivery pipe, a delivery handle fixedly connected to a proximal end of the delivery pipe, a sheath-core slidably inserted into the delivery pipe and a sheath-core handle fixedly connected to a proximal end of the sheath-core; wherein, a distal portion of the delivery pipe is a flexible pipe and a proximal portion of the delivery pipe is a rigid pipe; the sheath-core includes a tube having a cavity, a distal portion of the sheath-core is a flexible segment and a proximal portion of the sheath-core is a rigid segment; one or more pull threads are provided inside the cavity of the sheath-core; a distal end of each pull thread is fixedly connected to the distal end of the flexible segment of the sheath-core; a proximal end of each pull thread is connected to a drawing means arranged on the sheath-core handle, so as to drive the flexible segment at the distal end of the sheath-core to bend, and further to drive the distal end of the delivery pipe to bend.

2. The steerable cannula for delivering bone filler according to claim 1, wherein, the sheath-core has a single cavity or more cavities; in case of more cavities, one strengthening wire is provided inside one of the cavities; a proximal end of the strengthening wire is fixedly connected to the sheath-core handle.

3. The steerable cannula for delivering bone filler according to claim 1, wherein, the drawing means comprises a sliding groove arranged longitudinally along an axle direction of the sheath-core handle and a sliding block arranged in the sliding groove; the proximal end of each pull thread is connected to the sliding block; the pull thread is configured to be drawn through sliding the sliding block arranged in the sliding groove towards the proximal end, and the flexible segment of the distal end of the sheath-core is further driven to bend with a bending angle ranged from zero degree to 120 degrees.

4. The steerable cannula for delivering bone filler according to claim 3, wherein, a cover plate is fixedly attached to the distal end of the sheath-core handle; the cover plate is provided with a pull thread hole; and each pull thread goes through the pull thread hole and is fixedly connected to the sliding block.

5. The steerable cannula for delivering bone filler according to claim 1, wherein, the drawing means is a knob arranged on the sheath-core handle; the proximal end of each pull thread is fixedly connected to the knob; the pull thread is configured to be drawn by turning the knob, and the flexible segment at the distal end of the sheath-core is further driven to bend with a bending angle ranged from zero degree to 120 degrees.

6. The steerable cannula for delivering bone filler according to claim 1, wherein, the pull thread has a diameter ranged from 0.05 mm to 2 mm.

7. The steerable cannula for delivering bone filler according to claim 1, wherein, the distal end of the delivery pipe is closed; the distal end of the delivery pipe is provided with one or more holes at its side wall; the distal end of the delivery pipe is arc-shaped, flat-shaped or sharp-shaped; and the delivery handle is provided with a Luer joint for connecting a syringe of bone filler.

8. The steerable cannula for delivering bone filler according to claim 1, wherein, the distal end of the delivery pipe is open; the distal end of the sheath-core is closed, and the distal end of the sheath-core is arc-shaped, flat-shaped or sharp-shaped.

9. The steerable cannula for delivering bone filler according to claim 7 or claim 8, wherein, the distal end of the delivery pipe or the distal end of the sheath-core is sealed by a tapered metal head.

10. The steerable cannula for delivering bone filler according to claim 1, wherein, the flexible segment of the sheath-core is provided with several grooves at its tube wall.
